# EUROPEAN PATENT APPLICATION

(11) **EP 3 874 944 A1**
(43) Date of publication of application: **08.09.2021**
(21) Application number: 20160475.8
(22) Date of filing: 02.03.2020
(51) Int. Cl.: A01K 67/027, C12N 9/50, C12N 15/85

(54) **ANIMAL MODEL FOR HYPERKERATOSIS AND SEPSIS**

(71) Applicant: Christian-Albrechts-Universität zu Kiel, 24118 Kiel (DE)
(72) Inventor: Becker-Pauly, Christoph, 24229 Dänischenhagen (DE); Peters, Florian, 8005 Zürich (CH)
(74) Representative: Taruttis, Stefan Georg

(57) **Abstract**

Animal model for hyperkeratosis and/or for sepsis and/or for systemic inflammation, which animal has the advantage of developing hyperkeratosis and/or sepsis upon administration of an external stimulus at an age of the animal that can be chosen freely. The animal is e.g. for use in research, especially for use in processes for testing pharmaceutical compounds in respect of an effect on hyperkeratosis and/or on sepsis and/or on systemic inflammation.

## Description

The present invention relates to an animal model for hyperkeratosis and/or for sepsis and/or for systemic inflammation, which animal has the advantage of developing hyperkeratosis and/or sepsis upon administration of an external stimulus at an age of the animal that can be chosen freely. The animal is e.g. for use in research, especially for use in processes for testing pharmaceutical compounds in respect of an effect on hyperkeratosis and/or on sepsis and/or on systemic inflammation. The animal is a non-human mammal, e.g. a rodent, e.g. a mouse, rat, rabbit, guinea pig, or dog, cat, monkey, or a swine. Generally herein, the non-human mammal is also simply referred to as animal. The animal has the advantage that the sepsis and/or systemic inflammation is not elicited against an antigen, and hence the phenotype of sepsis and/or of systemic inflammation is not antigen-specific.

The animal of the invention is genetically manipulated to develop the phenotype of hyperkeratosis and/or sepsis upon activation by an external stimulus, which preferably is a synthetic compound. Accordingly, the invention also relates to a process for generating the animal model.

The animal of the invention has the advantage of reproducibly generating a phenotype of hyperkeratosis and/or of sepsis without administration of toxins or other pro-inflammatory stimuli and without mechanically destroying epithelia or tissues of the animal.

### State of the art

Stortz et al., ILAR Journal 90-105 (2017), review the advantages of using mice as animal models and describe murine sepsis models which are generated by exogenous administration of toxin or of a viable pathogen, or by puncture of intestines.

Holly et al., Kidney International 496-506 (2006), describe that upon induction of sepsis by puncture of intestines in rats, which resulted in acute kidney failure, changes of the concentration of a number of proteins in urine were found, the proteins including albumin, brush-border enzymes (e.g. meprin-1-alpha), and serine protease inhibitors. Meprin-1-alpha is mentioned as a potential biomarker and drug target.

Wang et al., SHOCK 141-147 (2011), describe that actinonin, a meprin-1-A inhibitor, should be evaluated as a therapeutic agent in targeting early and later organ-damaging effects of sepsis that was induced by puncturing the intestine subsequent to administration of actinonin.

### Object of the invention

It is an object of the invention to provide an animal model, which generates hyperkeratosis and/or systemic inflammation and/or sepsis upon induction of an external stimulus, which stimulus does not require the administration of a toxin nor of a pro-inflammatory molecule to the animal, nor mechanically damaging the animal model, e.g. no puncturing or other damaging of intestines. Preferably, the animal model is genetically manipulated such that administration of an external stimulus, which is a chemical compound, results in a phenotype of hyperkeratosis and/or of sepsis.

### Description of the invention

The invention achieves the object by the features of the claims and especially by providing a non-human animal, preferably a non-human mammal, which is genetically manipulated to overexpress meprin α under the control of an external stimulus. Meprin α can be overexpressed from an additional expression cassette that was introduced into the animal by genetic manipulation, or meprin α can be expressed from the endogenous meprin α encoding gene by genetic manipulation of its regulatory genetic elements. Accordingly, the animal is genetically manipulated to contain a genetic element which in the presence of an external stimulus induces overexpression of meprin α, e.g. from an expression cassette encoding meprin α or from the endogenous gene encoding meprin α. The meprin α can have the amino acid sequence of the endogenous meprin α, or the amino acid sequence of a heterologous meprin α.

The animal can be genetically manipulated to contain an expression cassette encoding meprin α under the control of an inducible promoter, which promoter is inducible by an external stimulus and which promoter in the absence of the external stimulus does not express meprin α. In the alternative or additionally, the animal can be genetically manipulated to contain an inducible promoter that is introduced in addition or replacing the original endogenous promoter of the natural meprin α gene, e.g. a promoter introduced by a CRISPR-mediated method, e.g. CRISPR-mediated gene enhancement (CRISPRa) targeting the endogenous meprin α gene. Accordingly, upon presence of the external stimulus, the promoter is activated to express meprin α. Generally, the expression of meprin α under the control of the promoter that is inducible by an external stimulus is also termed overexpression of meprin α, because this expression is in addition to and is independent of any endogenous expression of meprin α.

For introducing a promoter into a functional relationship to a meprin α encoding nucleic acid sequence, which encoding nucleic acid sequence can be one of the endogenous meprin α gene or a nucleic acid that is introduced along with the promoter, viral vectors can also be used.

Further, the invention provides a process for generating the animal which is genetically manipulated to contain an expression cassette encoding meprin α under the control of an inducible promoter, e.g. by genetically manipulating stem cells of the animal to contain an additional expression cassette for meprin α and/or an inducible promoter in addition to or replacing the endogenous promoter of the endogenous meprin α gene, and producing an animal from the genetically manipulated stem cells.

As the animal develops hyperkeratosis and/or sepsis and/or systemic inflammation upon administration of the external stimulus that induces the expression of meprin α from the expression cassette introduced by genetic manipulation, the invention further provides a process for investigating the effect of compounds, e.g. for screening compounds in search of pharmaceutically active compounds, by providing the animal, applying the external stimulus inducing the overexpression of meprin α from the expression cassette, and administering a compound prior to or subsequent to applying the external stimulus. This process makes use of the development of hyperkeratosis and/or sepsis and/or systemic inflammation in the animal upon administration of an external stimulus, which animal is genetically manipulated to comprise a promoter that is inducible by an external stimulus, and which promoter is functionally linked to a nucleic acid sequence encoding meprin α. As the overexpression of meprin α under the control of an inducible promoter by administration of the external stimulus that induces this promoter is sufficient for generating the phenotype of hyperkeratosis and/or sepsis and systemic inflammation, the process for generating this phenotype may consist of providing the genetically manipulated animal of the invention and administering the external stimulus, e.g. without administration of any additional toxin or other pro-inflammatory stimuli, and without mechanically destroying epithelia or tissues of the animal.

Surprisingly, it was found that overexpressing meprin α in an animal resulted in the generation of a phenotype, which resembles hyperkeratosis and/or sepsis and systemic inflammation. Herein, expression of meprin α that is in addition to normal expression of meprin α is also termed overexpression. This animal model, e.g. a mouse or rat, has shown the advantage of an apparently normal development, e.g. into an adult animal, and to develop hyperkeratosis and/or sepsis upon application of the external stimulus that induces the overexpression of meprin α, without administrating a toxin known to induce sepsis and without puncturing the intestine or otherwise surgically damaging the animal.

Preferably, the additional expression cassette and/or the endogenous meprin α gene is under the control of a promoter which upon presence of the external stimulus is activated at least in or only in keratinocytes, in hepatocytes, in immune cells, in endothelial cells, in enterocytes, in smooth muscle cells, and/or in kidney cells. Accordingly, the promoter can be a tissue-specific promoter, e.g. the KRT5 promoter for expression in keratinocytes, the Alb promoter or the LSEC promoter for expression in hepatocytes, the Csfr1 promoter or the LysM promoter for expression in immune cells, the Tie2 promoter for expression in endothelial cells, the villin promoter for expression in enterocytes, the Tagln promoter or the Myh11 promoter for expression in smooth muscle cells, or the Cdh16 promoter or the NPHS2 promoter for expression in kidney.

A preferred additional expression cassette encoding meprin α for introduction into the genome of the animal by genetic manipulation comprises the coding sequence of meprin α and a first promoter, which is inactive in the absence of an external stimulus, or which first promoter is arranged in combination with two recognition sites of a recombinase in a configuration in which the first promoter is inactive in respect of the meprin α coding sequence, and the animal further comprises a second expression cassette encoding the recombinase specific for the recognition sites under the control of an inducible promoter. In this embodiment, the second expression cassette forms the promoter inducible by an external stimulus, as the expression of the recombinase results in an activation of the expression cassette encoding meprin α. The two recognition sites can be arranged between the first promoter and the coding sequence for meprin α, and a stop element and optionally a selection marker can be arranged between the two recognition sites. Alternatively or additionally, the two recognition sites can be arranged to flank the first promoter or to flank the coding sequence of meprin α, so that the recombinase can convert the originally inactive arrangement of the promoter or of the coding sequence into an active arrangement, in which the first promoter drives expression of meprin α. The first promoter can be a strong constitutive promoter, e.g. the CAG promoter described by Miyazaki et al., Gene 79, 269-277 (1989). Generally, the recognition sites for a recombinase are arranged such that the recombinase can excise an element that otherwise prevents transcription of the meprin α encoding sequence, or the recognition sites can be arranged such that the recombinase turns a genetic element from an original inactive orientation into an active orientation that results in transcription of the meprin α encoding sequence.

In embodiments comprising a coding sequence for recombinase, the animal preferably contains the ERT2 system which in the presence of tamoxifen, e.g. for Cre recombinase, results in translocation of recombinase from the cytosol into the nucleus.

For a CRISPR-mediated induction of overexpression of meprin α from the endogenous meprin α gene using CRISPR-mediated gene enhancement, a guide RNA (gRNA) and a catalytically inactive Cas9 (dCas9) in combination with a transcription enhancer MP2-P65-HSF1 (MPH) is introduced into the animal, e.g. via viral particles, especially via adeno-associated viral particles.

Optionally, the animal is genetically manipulated to overexpress meprin α in cells which express a peptidase, e.g. at least one of trypsin, plasmin, neutrophil elastase, and/or preferably at least one kallikrein, especially preferred in cells which express KLK5, and/or RgpB. Accordingly, the genetic manipulation can be directed to at least one cell type expressing a peptidase, e.g. by genetic manipulation of the animal using e.g. viral vector particles which have preference for the at least one cell type. Alternatively, essentially all cells of the animal may be genetically manipulated for overexpression of meprin α at presence of an external stimulus only.

Optionally additionally, nucleic acid constructs for genetic manipulation can comprise an expression cassette encoding at least one peptidase, e.g. trypsin, plasmin, neutrophil elastase, and/or preferably at least one kallikrein, KLK5, and/or RgpB.

The invention is now described by way of examples and with reference to the figures, which show in
- Fig. 1 A) to C) schematic drawings of nucleic acid constructs,
- Fig. 2 A) a schematic drawing of a nucleic acid construct of the invention,
- Fig. 2 B) a schematic drawing of the time course of a process of the invention,
- Fig. 2 C) a graph of body weights of mice of the invention and of comparative mice,
- Fig. 3 A) a Western blot for overexpression of meprin α from a nucleic acid construct of the invention in mice,
- Fig. 3 B) a graph of the enzymatic activity of meprin α in skin samples of mice of the invention,
- Fig. 3 C) electron microscopic pictures of skin cross-section of a comparative mouse and of a mouse according to the invention,
- Fig. 3 D) a graph of the thickness of the epidermis of comparative mice and of mice according to the invention,
- Fig. 4 A) results of blood cell counts, and in
- Fig. 4 B) photographs of the inside of skin samples from comparative mice and from mice of the invention.

### Example: Generation of genetically manipulated mouse that develops hyperkeratosis and sepsis following administration of an external stimulus

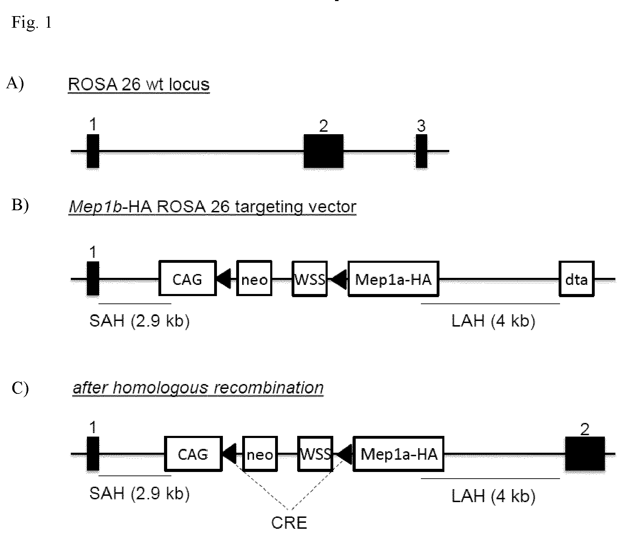

As an exemplary non-human mammal, mice (strain C57Bl/6N) were used for genetic manipulation to overexpress meprin α under the control of a promoter that is activated by an external stimulus. An expression cassette containing the cDNA of the murine meprin α coding sequence (Mepla) with a CAG promoter and between these, flanked by recombinase recognition sites, a stop element and the neomycin resistance gene as a selection marker, was inserted into the Rosa26 locus. The wild type ROSA26 locus expresses two transcripts with no significant open reading frames (ORF) and a third in antisense orientation, which putatively encodes a protein, and which is not affected by the insertions. Targeting to the ROSA26 locus is conveniently achieved by introducing the desired gene into the first intron of the locus. The Rosa26 locus, wild-type, is schematically shown in Fig. 1A), comprising the sites exon 1 (reference numeral 1 in Fig. 1), exon 2 (reference numeral 2 in Fig. 1) and exon 3 (reference numeral 3 in Fig. 1).

Fig. 1B) shows the nucleic acid construct containing the expression cassette comprising the element short arm of homology (SAH), the CAG promoter, a first recombinase recognition site (◄), the neomycin resistance gene (neo), Westphal stop sequence (WSS) as a stop element, a second recombinase recognition site (◄, loxP), the coding sequence for meprin α with a C-terminal HA tag (MEP1a-HA), long arm of homology (LAH), and diphtheria toxin fragment A (dta), is schematically shown.

Fig. 1C) schematically shows the nucleic acid construct containing this expression cassette after insertion into the genomic DNA by homologous recombination in the Rosa26 locus, and indicates the activity of Cre recombinase (CRE) to excise the resistance gene (neo) and the stop element (WSS) that are flanked by the recombinase recognition sites.

The nucleic acid sequence of the construct shown in Fig. 1B) is given as SEQ ID NO: 1.

The coding sequence of meprin α (nucleotides No. 4573 to 6852 of SEQ ID NO: 1) corresponds to the cDNA sequence of the homologous meprin α, in this case the mouse sequence. The coding sequence for meprin α contained an additional tag-sequence encoding HA at its 3'-terminus (nucleotides No. 6853 to 6906 of SEQ ID NO: 1), resulting in the meprin α comprising the amino acid sequence of SEQ ID NO: 2 with an additional C-terminal HA tag. The HA tag is derived from the human influenza hemagglutinin and has the amino acid sequence YPYDVPDYA (SEQ ID NO: 3). Upon cleavage of the primary translation product of meprin α by a proprotein convertase on the natural secretory pathway, the C-terminus including the HA tag is cleaved off and therefore is assumed to not affect the activity of the resulting active meprin α. However, the C-terminal tag can be used to distinguish the translation product originating from the expression cassette from endogenous meprin α. The nucleic acid construct comprised regions of homology to the murine Rosa26 locus at both its ends for integration into this genomic locus. As a reporter gene, the nucleic acid construct contained the coding sequence for enhanced green fluorescent protein (EGFP). Between the CAG promoter (nucleotides 989 to 1576 of SEQ ID NO: 1) and the coding sequence for meprin α, there is the Westphal stop sequence (WSS, nucleotides 3217 to 4514 of SEQ ID NO: 1) and a neomycin resistance as a selection marker (nucleotides 2116 to 2919 of SEQ ID NO: 1) flanked by recombinase recognition sites (LoxP, nucleotides 2021 to 2054 and nucleotides 4527 to 4560 of SEQ ID NO: 1).

The mice which contained the inserted nucleic acid construct in their Rosa26 locus were mated with Krt5-Cre driver mice (strain B6N.129S6(Cg)-Krt5tm1.1(cre/ERT2)Blh/J) that contain an expression cassette for recombinase Cre under the control of the keratin 5 promoter which is inducible by tamoxifen. In this embodiment, tamoxifen is the external stimulus, and the expression cassette for the recombinase forms the inducible promoter, as activity of the recombinase effects excision of the stop element, and hence the CAG promoter becomes active to translate meprin α. In addition, the mice contained the ERT2 system, which results in the translocation of cytosolic Cre into the nucleus upon presence of tamoxifen, which is preferable for time-dependent expression of meprin α in adult mice.

Recombinant mice that were identified to contain this nucleic acid construct were shown to exhibit a phenotype resembling sepsis and/or hyperkeratosis in 100% of the animals subsequent to administration of tamoxifen, but in the absence of tamoxifen these mice did not show any obvious phenotypic differences from the original strains.

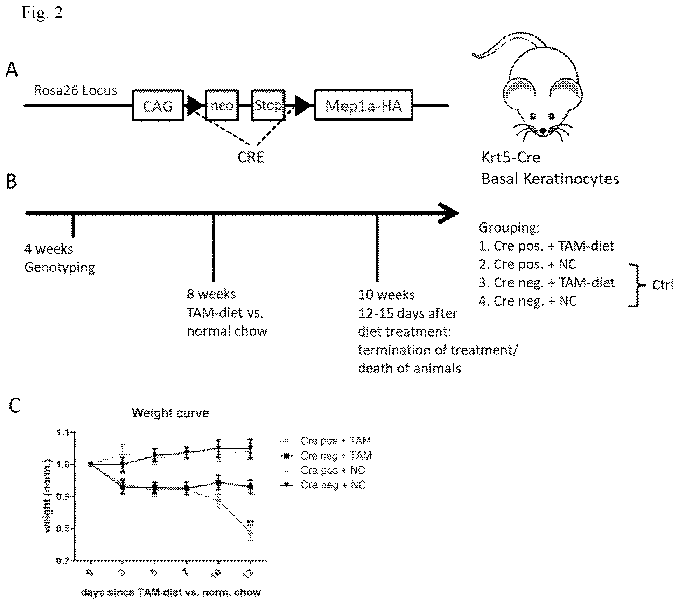

Fig. 2A) schematically shows a nucleic acid construct for overexpressing meprin α, which construct is integrated at the Rosa26 locus into the genome. Generally preferred, the recombinase recognition sites (►, loxP) flanking the stop element, which is excised by the recombinase (Cre) are arranged in the same orientation. The animal of the invention in addition to this nucleic acid construct for overexpressing meprin α contains an expression cassette for the cognate recombinase, in this embodiment under the control of a keratinocyte-specific promoter (Krt5-Cre).

A process for inducing sepsis and/or hyperkeratosis in the mice is shown in Fig. 2 B), with genotyping the mice at the age of 4 weeks, and at 8 weeks administering the external stimulus for overexpression of meprin α by feeding a tamoxifen-containing diet (TAM-diet), or normal feed without tamoxifen (normal chow, NC). For comparison, the genetically manipulated mice without being crossed with Krt5-Cre driver mice (Cre neg.) were treated with normal feed (NC) or with tamoxifen-containing diet (TAM-diet). None of the controls developed sepsis or hyperkeratosis. It was found that at 10 weeks, i.e. 12 to 15 days subsequent to the administration of tamoxifen, only the recombinant mice that are genetically manipulated for overexpression of meprin α according to the invention die from sepsis, or are euthanized after developing a state of sepsis.

Fig. 2C) shows the body weights determined for these animals (Cre pos + TAM: n = 7; Cre neg + TAM: n = 6; Cre pos + NC: n = 6; Cre neg + NC: n = 5 per group). It can be seen that presence of tamoxifen resulted in a weight loss, which gave a constant lower weight (10% weight loss compared to initial weight at week 8) also for the comparative mice which contained the nucleic acid construct comprising the expression cassette for meprin α but did not contain the inducible promoter formed by the expression cassette for recombinase Cre. Only the mice according to the invention suffered subsequent further weight loss (20% weight loss compared to the initial weight at week 8) at day 7 to 12, indicating the effects of sepsis. The control mice that were fed normal feed without tamoxifen as the external stimulus were not affected by the nucleic acid construct comprising the expression cassette for meprin α in combination with presence or absence of the expression cassette for recombinase Cre.

The expression of meprin α was tested in skin lysates of these mice by Western blotting, using an anti-HA antibody (C29F4 obtainable from Cell Signaling Technology) for detecting the HA-tag of meprin α originating from the nucleic acid construct comprising the expression cassette for meprin α. In short, skin was snap frozen in liquid nitrogen or directly pulverized in a nitrogen cooled mortar. Skin lysis buffer (50 mM Tris-HCl [pH 6.8], 150 mM NaCl, 1% [v/v] Triton X-100, 1% [w/v] SDS, 2 mM EDTA, Complete® Protease Inhibitor Cocktail [Roche]) was added to the skin powder and incubated for 24 h at 4°C under rolling conditions. Skin lysates were centrifuged at 13.000 rpm, 4°C for 30 min and transferred into new microcentrifuge tubes. Total protein concentration was measured by BCA assay and 50 µg of denatured skin lysates used for SDS-PAGE and Western Blot analysis.

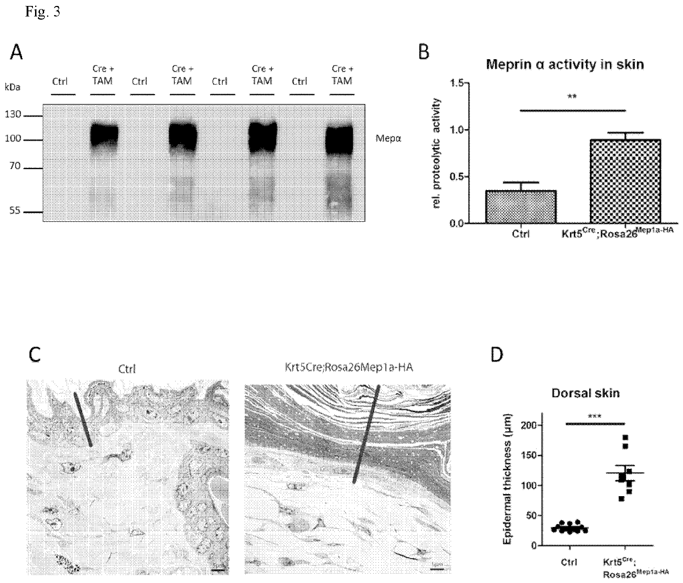

Fig. 3A) shows the Western blot and the band detected by the anti-HA antibody shows a molecular weight corresponding to that of meprin α. The activity of meprin α originating from the nucleic acid construct comprising the expression cassette for meprin α was determined by analysing the proteolytic activity in skin lysates. Fig. 2B) shows the increase of proteolytic activity in the skin samples of mice (Krt5^{Cre}, Ros26^{Mep1a-HA}) according to the invention in the presence of the external stimulus activating overexpression of meprin α, showing that the translation product of the nucleic acid construct comprising the expression cassette for meprin α generated enzymatically active meprin α.

Fig. 3C) shows pictures of transmission electron microscopy of skin samples of a comparative mouse (Ctrl) and of a mouse according to the invention (Krt5Cre;Rosa26Mepla-HA). This shows a significant thickening (indicated by inserted line) of the epidermis and by enhanced terminal differentiation of keratinocytes in the mouse which according to the invention is genetically manipulated to overexpress meprin α in the presence of an external stimulus. Fig. 3D) shows measurement results for the epidermal thickness of dorsal skin samples of comparative mice (Ctrl) and of mice according to the invention (Krt5^{Cre}; Rosa26^{Mep1a-HA}), indicating the development of hyperkeratosis by the significantly increased thickness of the epidermis in the mice according to the invention.

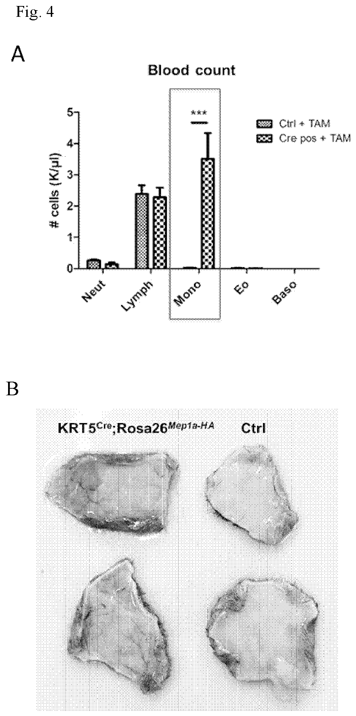

Fig. 4A) shows the number of blood cells (x 1000 cells /µl (K/µl)) from analyses at day 12 of the feed containing tamoxifen of comparative mice that contained the expression cassette for meprin α but no expression cassette for Cre (Ctrl + TAM, left columns) and for mice according to the invention overexpressing meprin α (Cre pos + TAM, right columns), in both cases with tamoxifen containing feed. The results for neutrophils (Neut), lymphocytes (Lymph), eosinophils (Eo), and basophils (Baso) show no significant influence of the overexpression of meprin α, but the concentration of monocytes (Mono) is drastically increased to 1850 to 5540 cells per µl in the mice of the invention overexpressing meprin α compared to 10 to 30 cells per µl in comparative mice. This indicates a phenotype of sepsis in the mice that overexpress meprin α.

Fig. 4B) shows photographs of the inner sides of excised skin samples originating from mice according to the invention, which overexpress meprin α (Krt5^{Cre}; Rosa26^{Mep1a-HA}) and from comparative mice (Ctrl). These photographs show that in the mice overexpressing meprin α, the blood vessels in the sub cutis have stronger permeability than those of comparative mice. It is currently assumed that the stronger permeability of blood vessels in the sub cutis are induced by interactions of the endothelium with monocytic cells.

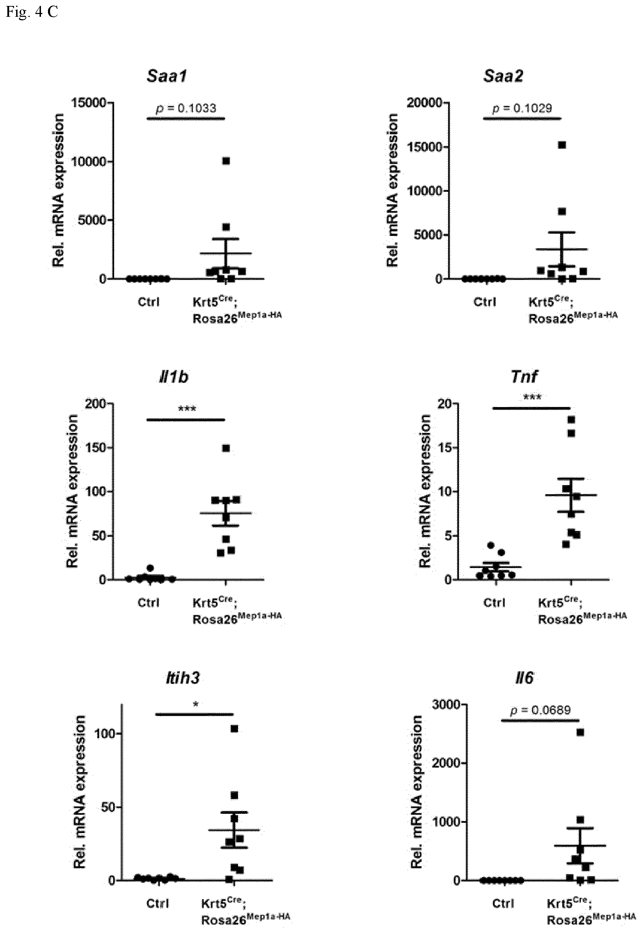

Fig. 4C) shows the results of RNA analyses of the acute-phase proteins and inflammatory cytokines. It was found that mRNA levels of Saa1 and of Saa2 in the mice according to the invention, which overexpress meprin α (Krt5^{Cre}; Rosa26^{Mep1a-HA}) were strongly increased, with individual values spreading by a factor of 10000 up to 15000 higher than in control mice that do not overexpress meprin α. The strong increase of levels of Saa1 and of Saa2 could indicate a more intense state of sepsis. Also for IL-6 (116), a much higher concentration was found in mice overexpressing meprin α, with the highest increase by a factor of 2500 compared to control mice. Also, genes for pro-inflammatory cytokines IL-1b (Il1b) and tumor necrosis factor (Tnf) as well as for acute phase protein Iith3 (Itih3) were significantly upregulated in mice overexpressing meprin α.

The log2 differences of protein concentrations were determined by mass spectrometry analyses for skin lysates from mice overexpressing meprin α to the protein concentrations for skin lysates from mice that were not manipulated to overexpress meprin α.

From alterations in the total proteins in mice overexpressing meprin α in comparison to mice without genetic manipulation to overexpress meprin α the acute-phase proteins alpha-1-acid glycoprotein 2, serum amyloid A-1, and serum amyloid A-2 were identified as 3 of the 7 most highly upregulated proteins. Other proteins that were found to be upregulated in the mice according to the invention were connected to skin damage and to proteolysis, like protein S100-A9, neutrophilic granule protein, and matrix metalloprotease-9. Decreased expression was found for suprabasin and metallothionein-4.

These results also indicate that the genetically manipulated animals of the invention which overexpress meprin α in the presence of an external stimulus generate a phenotype indicating sepsis.

The analytical results of monocytosis, of induction of pro-inflammatory cytokine expression, the weight loss and the sudden deaths of animals according to the invention indicated that the overexpression of meprin α in the animals according to the invention results in the development of acute inflammation responses, indicating sepsis.

## Claims

1. Non-human mammal that is genetically manipulated to express meprin α in the presence of an external stimulus.

2. Mammal according to claim 1, **characterized in that** it is genetically manipulated to contain a promoter inserted in functional relation to the endogenous meprin α encoding nucleic acid sequence, which promoter is inducible by presence of the external stimulus.

3. Mammal according to claim 1, **characterized in that** it is genetically manipulated to contain an additional expression cassette encoding meprin α under the control of a promoter which upon presence of the external stimulus is activated.

4. Mammal according to one of the preceding claims, **characterized in that** the expression cassette encoding meprin α contains a promoter that is cell-type specific for keratinocytes, for hepatocytes, for immune cells, for endothelial cells, for enterocytes, for smooth muscle cells, and/or for kidney cells.

5. Mammal according to one of the preceding claims, **characterized in that** the coding sequence for meprin α is separated from its promoter by an intermediate stop element which is flanked by recognition sites for a recombinase and **in that** the mammal contains an expression cassette for the cognate recombinase under the control of a promoter which is induced by the presence of the external stimulus.

6. Mammal according to one of the preceding claims, **characterized in that** it is genetically manipulated that the endogenous meprin α gene is under the control of a promoter which upon presence of the external stimulus is activated.

7. Mammal according to one of the preceding claims, **characterized in that** its endogenous gene encoding meprin α is genetically manipulated by its endogenous promoter being replaced by a promoter that is activated by the presence of the external stimulus.

8. Mammal according to one of the preceding claims, **characterized in that** it is in addition genetically manipulated to contain an expression cassette encoding a peptidase.

9. Process for testing chemical compounds, comprising providing a non-human mammal according to one of the preceding claims, administering the external stimulus to the animal for generating a phenotype of sepsis, systemic inflammation and/or of hyperkeratosis, and administering at least one chemical compound to the animal.

10. Process according to claim 9, **characterized in that** no additional toxin or proinflammatory compound is administered to the animal and no mechanical damage is inflicted upon the animal.

11. Process according to one of claims 9 to 10, **characterized in that** meprin α is expressed in keratinocytes, in hepatocytes, in immune cells, in endothelial cells, in enterocytes, in smooth muscle cells, and/or in kidney cells.

12. Process for generating a non-human animal which develops a phenotype of sepsis and/or of hyperkeratosis and/or of systemic inflammation, **characterized by** introducing into the animal a genetic manipulation that results in the presence of a promoter which is inducible by presence of an external stimulus and which induces overexpression of meprin α.

13. Process for generating a phenotype of sepsis and/or of hyperkeratosis and/or of systemic inflammation in a non-human mammal, comprising the steps of providing a non-human mammal according to one of claims 1 to 8, and administering an external stimulus to the animal.


SEQUENCE LISTING

<110> Christian-Albrechts-Universität zu Kiel

<120> Animal model for hyperkeratosis and sepsis

<130> K1039EP

<160> 4

<170> BiSSAP 1.3.6

<210> 1
<211> 9150
<212> DNA
<213> Artificial Sequence


<220>
<223> nucleic acid construct for for overexpressing meprin

<220>
<221> misc_feature
<222> 1..22
<223> /note="forward primer for genotyping"

<220>
<221> misc_feature
<222> 1..204
<223> /note="sequence of homology to Rosa26 locus"

<220>
<221> misc_feature
<222> 366..387
<223> /note="reverse primer for genotyping"

<220>
<221> promoter
<222> 989..1576
<223> /note="CAG promoter"

<220>
<221> misc_recomb
<222> 2021..2054
<223> /note="LoxP site"

<220>
<221> misc_feature
<222> 2116..2919
<223> /note="neomycin resistance"

<220>
<221> misc_feature
<222> 2117..4514
<223> /note="Westphal stop sequence (STOP cassette) "

<220>
<221> misc_recomb
<222> 4527..4560
<223> /note="LoxP site"

<220>
<221> CDS

<222> 4573..6852
<223> /product="meprin "
      /transl_table=1

<220>
<221> CDS
<222> 6853..6906
<223> /product="HA tag"
      /transl_table=1

<220>
<221> CDS
<222> 7590..8309
<223> /product="EGFP"
      /transl_table=1

<220>
<221> misc_feature
<222> 8757..9150
<223> /note="sequence of homology to Rosa26 locus"

<220>
<221> misc_feature
<222> 9120..9140
<223> /note="reverse primer for genotyping"

<400> 1
aaagtcgctc tgagttgtta tcagtaaggg agctgcagtg gagtaggcgg ggagaaggcc    60

gcacccttct ccggaggggg gaggggagtg ttgcaatacc tttctgggag ttctctgctg   120

cctcctggct tctgaggacc gccctgggcc tgggagaatc ccttccccct cttccctcgt   180

gatctgcaac tccagtcttt ctagcatctg tagggcgcag tagtccaggg tttccttgat   240

gatgtcatac ttatcctgtc cctttttttt ccacagctcg cggttgagga caaactcttc   300

gcggtctttc cagtggttaa ttaaagttat aatcgctgag gtaatattta aaatcatttt   360

caaatgattc acagttaatt tgcgacaata taattttatt ttcacataaa ctagacgcct   420

tgtcgtcttc ttcttcgtat tccttctctt tttcattttt ctcttcataa aaattaacat   480

agttattatc gtatccatat atgtatctat cgtatagagt aaattttttg ttgtcataaa   540

tatatatgtc ttttttaatg gggtgtatag taccgctgcg catagttttt ctgtaattta   600

caacagtgct attttctggt agttcttcgg agtgtgttgc tttaattatt aaatttatat   660

aatcaatgaa tttgggatcg tcggttttgt acaatatgtt gccggcatag tacgcagctt   720

cttctagttc aattacacca ttttttagca gcaccggatt aacataactt tccaaaatgt   780

tgtacgaacc gttaaacaaa aacagttcac ctcccttttc tatactattg tctgcgagca   840

gttgtttgtt gttaaaaata acagccattg taatgagacg cacaaactaa tatcacaaac   900

tggaaatgtc tatcaatata tagttgctct agttattaat agtaatcaat tacggggtca   960

ttagttcata gcccatatat ggagttccgc gttacataac ttacggtaaa tggcccgcct  1020

ggctgaccgc ccaacgaccc ccgcccattg acgtcaataa tgacgtatgt tcccatagta  1080

acgccaatag ggactttcca ttgacgtcaa tgggtggact atttacggta aactgcccac 1140

ttggcagtac atcaagtgta tcatatgcca agtacgcccc ctattgacgt caatgacggt 1200

aaatggcccg cctggcatta tgcccagtac atgaccttat gggactttcc tacttggcag 1260

tacatctacg tattagtcat cgctattacc atgcatggtc gaggtgagcc ccacgttctg 1320

cttcactctc cccatctccc ccccctcccc acccccaatt ttgtatttat ttatttttta 1380

attattttgt gcagcgatgg gggcgggggg gggggggggg cgcgcgccag gcggggcggg 1440

gcggggcgag gggcggggcg gggcgaggcg gagaggtgcg gcggcagcca atcagagcgg 1500

cgcgctccga aagtttcctt ttatggcgag gcggcggcgg cggcggccct ataaaaagcg 1560

aagcgcgcgg cgggcgggag tcgctgcgac gctgccttcg ccccgtgccc cgctccgccg 1620

ccgcctcgcg ccgcccgccc cggctctgac tgaccgcgtt actcccacag gtgagcgggc 1680

gggacggccc ttctccttcg ggctgtaatt agcgcttggt ttaatgacgg cttgtttctt 1740

ttctgtggct gcgtgaaagc cttgaggggc tccgggaggg ccctttgtgc ggggggagcg 1800

gctcggggct gtccgcgggg ggacggctgc cttcgggggg gacggggcag ggcggggttc 1860

ggcttctggc gtgtgaccgg cggctctaga gcctcgctag catctgtagg gcgcagtagt 1920

ccagggtttc cttgatgatg tcatacttat cctgtccctt ttttttccac agctcgcggt 1980

tgaggacaaa ctcttcgcgg tctttccagt ggttaattaa ataacttcgt atagcataca 2040

ttatacgaag ttatggatcc gaacaaacga cccaacaccc gtgcgtttta ttctgtcttt 2100

ttattgccga tcccctcaga agaactcgtc aagaaggcga tagaaggcga tgcgctgcga 2160

atcgggagcg gcgataccgt aaagcacgag gaagcggtca gcccattcgc cgccaagctc 2220

ttcagcaata tcacgggtag ccaacgctat gtcctgatag cggtccgcca cacccagccg 2280

gccacagtcg atgaatccag aaaagcggcc attttccacc atgatattcg gcaagcaggc 2340

atcgccatgg gtcacgacga gatcctcgcc gtcgggcatg cgcgccttga gcctggcgaa 2400

cagttcggct ggcgcgagcc cctgatgctc ttcgtccaga tcatcctgat cgacaagacc 2460

ggcttccatc cgagtacgtg ctcgctcgat gcgatgtttc gcttggtggt cgaatgggca 2520

ggtagccgga tcaagcgtat gcagccgccg cattgcatca gccatgatgg atactttctc 2580

ggcaggagca aggtgagatg acaggagatc ctgccccggc acttcgccca atagcagcca 2640

gtcccttccc gcttcagtga caacgtcgag cacagctgcg caaggaacgc ccgtcgtggc 2700

cagccacgat agccgcgctg cctcgtcctg cagttcattc agggcaccgg acaggtcggt 2760

cttgacaaaa agaaccgggc gcccctgcgc tgacagccgg aacacggcgg catcagagca 2820

gccgattgtc tgttgtgccc agtcatagcc gaatagcctc tccacccaag cggccggaga 2880

acctgcgtgc aatccatctt gttcaatggc cgatcccata ttggctgcag ggtcgctcgg 2940

tgttcgaggc cacacgcgtc accttaatat gcgaagtgga cctgggaccg cgccgccccg 3000

actgcatctg cgtgttcgaa ttcgccaatg acaagacgct gggcggggtt tgctcgacat 3060

tgggtggaaa cattccaggc ctgggtggag aggctttttg cttcctcttg caaaaccaca 3120

ctgctcgaca ttgggtggaa acattccagg cctgggtgga gaggcttttt gcttcctctt 3180

gcaaaaccac actgctcgac tagtgattaa agtcgactcg gggacaccaa atatggcgat 3240

ctcggccttt tcgtttcttg gagctgggac atgtttgcca tcgatccatc taccaccaga 3300

acggccgtta gatctgctgc caccgttgtt tccaccgaag aaaccaccgt tgccgtaacc 3360

accacgacgg ttgttgctaa agaagctgcc accgccacgg ccaccgttgt agccgccgtt 3420

gttgttattg tagttgctca tgttatttct ggcacttctt ggttttcctc ttaagtgagg 3480

aggaacataa ccattctcgt tgttgtcgtt gatgcttaaa ttttgcactt gttcgctcag 3540

ttcagccata atatgaaatg cttttcttgt tgttcttacg gaataccact tgccacctat 3600

caccacaact aactttttcc cgttcctcca tctcttttat attttttttc tcgagggatc 3660

tttgtgaagg aaccttactt ctgtggtgtg acataattgg acaaactacc tacagagatt 3720

taaagctcta aggtaaatat aaaattttta agtgtataat gtgttaaact actgattcta 3780

attgtttgtg tattttagat tccaacctat ggaactgatg aatgggagca gtggtggaat 3840

gcctttaatg aggaaaacct gttttgctca gaagaaatgc catctagtga tgatgaggct 3900

actgctgact ctcaacattc tactcctcca aaaaagaaga gaaaggtaga agaccccaag 3960

gactttcctt cagaattgct aagttttttg agtcatgctg tgtttagtaa tagaactctt 4020

gcttgctttg ctatttacac cacaaaggaa aaagctgcac tgctatacaa gaaaattatg 4080

gaaaaatatt ctgtaacctt tataagtagg cataacagtt ataatcataa catactgttt 4140

tttcttactc cacacaggca tagagtgtct gctattaata actatgctca aaaattgtgt 4200

acctttagct ttttaatttg taaaggggtt aataaggaat atttgatgta tagtgccttg 4260

actagagatc ataatcagcc ataccacatt tgtagaggtt ttacttgctt taaaaaacct 4320

cccacacctc cccctgaacc tgaaacataa aatgaatgca attgttgttg ttaacttgtt 4380

tattgcagct tataatggtt acaaataaag caatagcatc acaaatttca caaataaagc 4440

atttttttca ctgcattcta gttgtggttt gtccaaactc atcaatgtat cttatcatgt 4500

ctggatctga catggtaagt aagcttataa cttcgtatag catacattat acgaagttat 4560

cggcgcgcca cc atg gca agg agg ctt ggc aga tcc agt tca ttt gca ata 4611
Met Ala Arg Arg Leu Gly Arg Ser Ser Ser Phe Ala Ile
1               5                   10

atg ctt tgg ata caa cct gcg tgt ctt ctc tca ctg att ttt tct gcc 4659
Met Leu Trp Ile Gln Pro Ala Cys Leu Leu Ser Leu Ile Phe Ser Ala
    15                  20                  25

cac ata gca gct gta tcg att aag cat ctt ttg aat gga tct gat cac 4707
His Ile Ala Ala Val Ser Ile Lys His Leu Leu Asn Gly Ser Asp His

30                  35                  40                  45

gat aca gat gtt ggt gaa cag aag gat att ttc gaa atc aat tta gct     4755
Asp Thr Asp Val Gly Glu Gln Lys Asp Ile Phe Glu Ile Asn Leu Ala
                50                  55                  60

gca ggc ctg aac ctc ttt caa ggg gac atc ctt ctg ccc aga acc aga     4803
Ala Gly Leu Asn Leu Phe Gln Gly Asp Ile Leu Leu Pro Arg Thr Arg
            65                  70                  75

aat gcc atg cga gat ccc tca agc aga tgg aag ctc ccc att cca tac     4851
Asn Ala Met Arg Asp Pro Ser Ser Arg Trp Lys Leu Pro Ile Pro Tyr
        80                  85                  90

att ctg gct gac aat ctg gag ctg aat gcc aaa gga gcc atc ctc cat     4899
Ile Leu Ala Asp Asn Leu Glu Leu Asn Ala Lys Gly Ala Ile Leu His
    95                  100                 105

gcc ttt gag atg ttc cgc ctc aag tct tgt gtg gat ttc aag ccc tac     4947
Ala Phe Glu Met Phe Arg Leu Lys Ser Cys Val Asp Phe Lys Pro Tyr
110                 115                 120                 125

gaa ggg gag agt tcg tat atc atc ttc cag aaa ctt tct ggg tgc tgg     4995
Glu Gly Glu Ser Ser Tyr Ile Ile Phe Gln Lys Leu Ser Gly Cys Trp
                130                 135                 140

tct atg att ggt gat caa cag gtg gga cag aac att tcc att ggt gag     5043
Ser Met Ile Gly Asp Gln Gln Val Gly Gln Asn Ile Ser Ile Gly Glu
            145                 150                 155

gga tgt gac ttt aag gcc acc att gaa cat gaa atc ctg cat gct ctg     5091
Gly Cys Asp Phe Lys Ala Thr Ile Glu His Glu Ile Leu His Ala Leu
        160                 165                 170

gga ttc ttc cat gag cag tca agg act gac cgg gat gat tat gtg aac     5139
Gly Phe Phe His Glu Gln Ser Arg Thr Asp Arg Asp Asp Tyr Val Asn
    175                 180                 185

att tgg tgg gat caa atc att aca gac tat gaa cat aat ttc aac aca     5187
Ile Trp Trp Asp Gln Ile Ile Thr Asp Tyr Glu His Asn Phe Asn Thr
190                 195                 200                 205

tat gac gac aac acc atc aca gac ctc aac aca ccc tat gat tat gag     5235
Tyr Asp Asp Asn Thr Ile Thr Asp Leu Asn Thr Pro Tyr Asp Tyr Glu
                210                 215                 220

tcc ctg atg cac tac ggg cca ttt tcg ttt aac aag aac gaa agc atc     5283
Ser Leu Met His Tyr Gly Pro Phe Ser Phe Asn Lys Asn Glu Ser Ile
            225                 230                 235

cct acc att act acc aag atc cct gag ttt aac acc atc att gga caa     5331
Pro Thr Ile Thr Thr Lys Ile Pro Glu Phe Asn Thr Ile Ile Gly Gln
        240                 245                 250

ctc ccg gac ttc agt gcc att gat cta ata aga ctg aat cga atg tac     5379
Leu Pro Asp Phe Ser Ala Ile Asp Leu Ile Arg Leu Asn Arg Met Tyr
    255                 260                 265

aac tgc acc gca aca cat act ctg ttg gac cac tgt gac ttt gag aag     5427
Asn Cys Thr Ala Thr His Thr Leu Leu Asp His Cys Asp Phe Glu Lys
270                 275                 280                 285

acc aac gtc tgt ggg atg atc cag gga acc aga gat gat gct gat tgg     5475

Thr Asn Val Cys Gly Met Ile Gln Gly Thr Arg Asp Asp Ala Asp Trp
                290                 295                 300

gct cat ggg gac agc agt cag cct gaa caa gtg gat cac acc ttg gtg     5523
Ala His Gly Asp Ser Ser Gln Pro Glu Gln Val Asp His Thr Leu Val
            305                 310                 315

gga cag tgt aaa ggt gcc ggc tac ttc atg ttc ttc aat acc agc ttg     5571
Gly Gln Cys Lys Gly Ala Gly Tyr Phe Met Phe Phe Asn Thr Ser Leu
        320                 325                 330

ggg gca aga gga gag gca gcc ctt ctg gag tct cga atc ctt tat ccc     5619
Gly Ala Arg Gly Glu Ala Ala Leu Leu Glu Ser Arg Ile Leu Tyr Pro
    335                 340                 345

aag agg aag cag cag tgt ttg cag ttt ttt tat aag atg act gga agc     5667
Lys Arg Lys Gln Gln Cys Leu Gln Phe Phe Tyr Lys Met Thr Gly Ser
350                 355                 360                 365

cct gca gac aga ttt gaa gtc tgg gtg aga agg gac gac aac gcg ggc     5715
Pro Ala Asp Arg Phe Glu Val Trp Val Arg Arg Asp Asp Asn Ala Gly
                370                 375                 380

aag gtg cga cag ctg gcc aag atc cag acc ttt caa gga gac tct gat     5763
Lys Val Arg Gln Leu Ala Lys Ile Gln Thr Phe Gln Gly Asp Ser Asp
            385                 390                 395

cac aac tgg aaa att gct cat gtg aca ctc aat gaa gaa aag aaa ttt     5811
His Asn Trp Lys Ile Ala His Val Thr Leu Asn Glu Glu Lys Lys Phe
        400                 405                 410

cgc tat gtt ttc ctg ggt acc aaa ggg gac cct ggt aac tca agt ggt     5859
Arg Tyr Val Phe Leu Gly Thr Lys Gly Asp Pro Gly Asn Ser Ser Gly
    415                 420                 425

ggt att tac ctt gat gac atc act ctg aca gaa acc ccc tgc cct gca     5907
Gly Ile Tyr Leu Asp Asp Ile Thr Leu Thr Glu Thr Pro Cys Pro Ala
430                 435                 440                 445

ggg gtt tgg acc atc cgg aat atc tcc cag atc ctt gag aac aca gtg     5955
Gly Val Trp Thr Ile Arg Asn Ile Ser Gln Ile Leu Glu Asn Thr Val
                450                 455                 460

aaa ggg gac aag ctc gtg agc cct cgg ttc tac aat tct gag ggt tac     6003
Lys Gly Asp Lys Leu Val Ser Pro Arg Phe Tyr Asn Ser Glu Gly Tyr
            465                 470                 475

ggt gtg ggg gtg acc ttg tac ccc aat ggt aga atc acc agt aac tct     6051
Gly Val Gly Val Thr Leu Tyr Pro Asn Gly Arg Ile Thr Ser Asn Ser
        480                 485                 490

ggc ttc ctg gga ctc act ttt cac ttg tac agt ggg gat aac gat gcg     6099
Gly Phe Leu Gly Leu Thr Phe His Leu Tyr Ser Gly Asp Asn Asp Ala
    495                 500                 505

att ctg gag tgg cca gtg gaa aac aga cag gcc ata atg acc atc ctg     6147
Ile Leu Glu Trp Pro Val Glu Asn Arg Gln Ala Ile Met Thr Ile Leu
510                 515                 520                 525

gac cag gaa gct gat acc agg aac agg atg tcc ctg acc ttg atg ttc     6195
Asp Gln Glu Ala Asp Thr Arg Asn Arg Met Ser Leu Thr Leu Met Phe
                530                 535                 540

act acc tcc aag aac caa aca tct tca gct ata aat ggc tct gtc atc     6243
Thr Thr Ser Lys Asn Gln Thr Ser Ser Ala Ile Asn Gly Ser Val Ile
            545                 550                 555

tgg gac agg cca tcc aag gtg gga gtc tat gat aag gac tgt gat tgt     6291
Trp Asp Arg Pro Ser Lys Val Gly Val Tyr Asp Lys Asp Cys Asp Cys
        560                 565                 570

ttc aga agc ctt gac tgg ggc tgg ggt cag gcc atc tcc cac cag ctg     6339
Phe Arg Ser Leu Asp Trp Gly Trp Gly Gln Ala Ile Ser His Gln Leu
    575                 580                 585

ctg aag aga aga aat ttt ctt aaa ggc gac agc ctc atc att ttc gtg     6387
Leu Lys Arg Arg Asn Phe Leu Lys Gly Asp Ser Leu Ile Ile Phe Val
590                 595                 600                 605

gac ttt aaa gac ctc acc cac ctg aac cgg act gaa gtt cca gct tca     6435
Asp Phe Lys Asp Leu Thr His Leu Asn Arg Thr Glu Val Pro Ala Ser
                610                 615                 620

gcc aga agc acg atg ccc aga ggc ctc ctt ctg caa ggc cag gag tct     6483
Ala Arg Ser Thr Met Pro Arg Gly Leu Leu Leu Gln Gly Gln Glu Ser
            625                 630                 635

cct gcc ttg gga gaa agc tcc aga aaa gcc atg ctg gag gaa tcc ctg     6531
Pro Ala Leu Gly Glu Ser Ser Arg Lys Ala Met Leu Glu Glu Ser Leu
        640                 645                 650

cca agc agc ctg ggc cag aga cat ccc agc aga cag aag cgc tcg gtg     6579
Pro Ser Ser Leu Gly Gln Arg His Pro Ser Arg Gln Lys Arg Ser Val
    655                 660                 665

gag aac act ggc ccc atg gag gac cac aac tgg cca cag tac ttc aga     6627
Glu Asn Thr Gly Pro Met Glu Asp His Asn Trp Pro Gln Tyr Phe Arg
670                 675                 680                 685

gac ccc tgt gac cca aac cct tgc cag aat gaa ggc acc tgt gtg aac     6675
Asp Pro Cys Asp Pro Asn Pro Cys Gln Asn Glu Gly Thr Cys Val Asn
                690                 695                 700

gta aag gga atg gcc agc tgc agg tgc gtc tct ggg cac gcc ttt ttc     6723
Val Lys Gly Met Ala Ser Cys Arg Cys Val Ser Gly His Ala Phe Phe
            705                 710                 715

tac gca ggg gag cgc tgt cag gcc atg cac gtg cac ggc agc ttg ctg     6771
Tyr Ala Gly Glu Arg Cys Gln Ala Met His Val His Gly Ser Leu Leu
        720                 725                 730

ggc ctg ctc att ggc tgc atc gct ggc ttg atc ttc ttg acc ttc gtc     6819
Gly Leu Leu Ile Gly Cys Ile Ala Gly Leu Ile Phe Leu Thr Phe Val
    735                 740                 745

aca ttc tct acc acc aac gga aag ctg cgg cag tac cca tac gac gtc     6867
Thr Phe Ser Thr Thr Asn Gly Lys Leu Arg Gln Tyr Pro Tyr Asp Val
750                 755                 760                 765

cca gac tac gct tac cca tac gac gtc cca gac tac gct tgaggcgcgc     6916
Pro Asp Tyr Ala Tyr Pro Tyr Asp Val Pro Asp Tyr Ala
                770                 775

cgcggccgcg atcaattcgg taccgaagtt cctattccga agttcctatt ctctagaaag     6976

tataggaact tcctcgaggg taccccaatt ccgccccccc ccccccccta acgttactgg     7036

ccgaagccgc ttggaataag gccggtgtgc gtttgtctat atgttatttt ccaccatatt 7096

gccgtctttt ggcaatgtga gggcccggaa acctggccct gtcttcttga cgagcattcc 7156

taggggtctt tcccctctcg ccaaaggaat gcaaggtctg ttgaatgtcg tgaaggaagc 7216

agttcctctg gaagcttctt gaagacaaac aacgtctgta gcgacccttt gcaggcagcg 7276

gaacccccca cctggcgaca ggtgcctctg cggccaaaag ccacgtgtat aagatacacc 7336

tgcaaaggcg gcacaacccc agtgccacgt tgtgagttgg atagttgtgg aaagagtcaa 7396

atggctctcc tcaagcgtat tcaacaaggg gctgaaggat gcccagaagg taccccattg 7456

tatgggatct gatctggggc ctcggtgcac atgctttaca tgtgtttagt cgaggttaaa 7516

aaacgtctag gccccccgaa ccacggggac gtggttttcc tttgaaaaac acgatgataa 7576

tatggccaca acc atg gtg agc aag ggc gag gag ctg ttc acc ggg gtg 7625
Met Val Ser Lys Gly Glu Glu Leu Phe Thr Gly Val
    780                 785                 790

gtg ccc atc ctg gtc gag ctg gac ggc gac gta aac ggc cac aag ttc 7673
Val Pro Ile Leu Val Glu Leu Asp Gly Asp Val Asn Gly His Lys Phe
                795                 800                 805

agc gtg tcc ggc gag ggc gag ggc gat gcc acc tac ggc aag ctg acc 7721
Ser Val Ser Gly Glu Gly Glu Gly Asp Ala Thr Tyr Gly Lys Leu Thr
            810                 815                 820

ctg aag ttc atc tgc acc acc ggc aag ctg ccc gtg ccc tgg ccc acc 7769
Leu Lys Phe Ile Cys Thr Thr Gly Lys Leu Pro Val Pro Trp Pro Thr
        825                 830                 835

ctc gtg acc acc ctg acc tac ggc gtg cag tgc ttc agc cgc tac ccc 7817
Leu Val Thr Thr Leu Thr Tyr Gly Val Gln Cys Phe Ser Arg Tyr Pro
    840                 845                 850

gac cac atg aag cag cac gac ttc ttc aag tcc gcc atg ccc gaa ggc 7865
Asp His Met Lys Gln His Asp Phe Phe Lys Ser Ala Met Pro Glu Gly
855                 860                 865                 870

tac gtc cag gag cgc acc atc ttc ttc aag gac gac ggc aac tac aag 7913
Tyr Val Gln Glu Arg Thr Ile Phe Phe Lys Asp Asp Gly Asn Tyr Lys
                875                 880                 885

acc cgc gcc gag gtg aag ttc gag ggc gac acc ctg gtg aac cgc atc 7961
Thr Arg Ala Glu Val Lys Phe Glu Gly Asp Thr Leu Val Asn Arg Ile
            890                 895                 900

gag ctg aag ggc atc gac ttc aag gag gac ggc aac atc ctg ggg cac 8009
Glu Leu Lys Gly Ile Asp Phe Lys Glu Asp Gly Asn Ile Leu Gly His
        905                 910                 915

aag ctg gag tac aac tac aac agc cac aac gtc tat atc atg gcc gac 8057
Lys Leu Glu Tyr Asn Tyr Asn Ser His Asn Val Tyr Ile Met Ala Asp
    920                 925                 930

aag cag aag aac ggc atc aag gtg aac ttc aag atc cgc cac aac atc 8105
Lys Gln Lys Asn Gly Ile Lys Val Asn Phe Lys Ile Arg His Asn Ile
935                 940                 945                 950

gag gac ggc agc gtg cag ctc gcc gac cac tac cag cag aac acc ccc     8153
Glu Asp Gly Ser Val Gln Leu Ala Asp His Tyr Gln Gln Asn Thr Pro
                955                 960                 965

atc ggc gac ggc ccc gtg ctg ctg ccc gac aac cac tac ctg agc acc     8201
Ile Gly Asp Gly Pro Val Leu Leu Pro Asp Asn His Tyr Leu Ser Thr
            970                 975                 980

cag tcc gcc ctg agc aaa gac ccc aac gag aag cgc gat cac atg gtc     8249
Gln Ser Ala Leu Ser Lys Asp Pro Asn Glu Lys Arg Asp His Met Val
        985                 990                 995

ctg ctg gag ttc gtg acc gcc gcc ggg atc act ctc ggc atg gac gag     8297
Leu Leu Glu Phe Val Thr Ala Ala Gly Ile Thr Leu Gly Met Asp Glu
    1000                1005                1010

ctg tac aag taa tccgggatcc ggagagctcc caacgaagtt cctattccga         8349
Leu Tyr Lys
1015

agttcctatt ctctagaaag tataggaact tcctcgaggt tggatgcagc ccgggggatc  8409

cactagttct agagcggccg atcagcctcg actgtgcctt ctagttgcca gccatctgtt  8469

gtttgcccct cccccgtgcc ttccttgacc ctggaaggtg ccactcccac tgtcctttcc  8529

taataaaatg aggaaattgc atcgcattgt ctgagtaggt gtcattctat tctggggggt  8589

ggggtggggc aggacagcaa gggggaggat tgggaagaca atagcaggca tgctggggat  8649

gcggtgggct ctatggcttc tgaggcggaa agaaccagct ggggctcgag atccactagt  8709

tctagcctcg aggctagagc ggccgccgcg gatatcgaat tcgctagaag atgggcggga  8769

gtcttctggg caggcttaaa ggctaacctg gtgtgtgggc gttgtcctgc aggggaattg  8829

aacaggtgta aaattggagg gacaagactt cccacagatt ttcggttttg tcgggaagtt  8889

ttttaatagg ggcaaataag gaaaatggga ggataggtag tcatctgggg ttttatgcag  8949

caaaactaca ggttattatt gcttgtgatc cgcctcggag tattttccat cgaggtagat  9009

taaagacatg ctcacccgag ttttatactc tcctgcttga gatccttact acagtatgaa  9069

attacagtgt cgcgagttag actatgtaag cagaatttta atcattttta aagagcccag  9129

tacttcatat ccatttctcc c                                            9150

<210> 2
<211> 760
<212> PRT
<213> Artificial Sequence


<220>
<223> [CDS]:4573..6852 from SEQ ID NO 1

<400> 2
Met Ala Arg Arg Leu Gly Arg Ser Ser Ser Phe Ala Ile Met Leu Trp
1               5                   10                  15
Ile Gln Pro Ala Cys Leu Leu Ser Leu Ile Phe Ser Ala His Ile Ala
            20                  25                  30
Ala Val Ser Ile Lys His Leu Leu Asn Gly Ser Asp His Asp Thr Asp

35                  40                  45
Val Gly Glu Gln Lys Asp Ile Phe Glu Ile Asn Leu Ala Ala Gly Leu
    50                  55                  60
Asn Leu Phe Gln Gly Asp Ile Leu Leu Pro Arg Thr Arg Asn Ala Met
65                  70                  75                  80
Arg Asp Pro Ser Ser Arg Trp Lys Leu Pro Ile Pro Tyr Ile Leu Ala
                85                  90                  95
Asp Asn Leu Glu Leu Asn Ala Lys Gly Ala Ile Leu His Ala Phe Glu
            100                 105                 110
Met Phe Arg Leu Lys Ser Cys Val Asp Phe Lys Pro Tyr Glu Gly Glu
        115                 120                 125
Ser Ser Tyr Ile Ile Phe Gln Lys Leu Ser Gly Cys Trp Ser Met Ile
    130                 135                 140
Gly Asp Gln Gln Val Gly Gln Asn Ile Ser Ile Gly Glu Gly Cys Asp
145                 150                 155                 160
Phe Lys Ala Thr Ile Glu His Glu Ile Leu His Ala Leu Gly Phe Phe
                165                 170                 175
His Glu Gln Ser Arg Thr Asp Arg Asp Asp Tyr Val Asn Ile Trp Trp
            180                 185                 190
Asp Gln Ile Ile Thr Asp Tyr Glu His Asn Phe Asn Thr Tyr Asp Asp
        195                 200                 205
Asn Thr Ile Thr Asp Leu Asn Thr Pro Tyr Asp Tyr Glu Ser Leu Met
    210                 215                 220
His Tyr Gly Pro Phe Ser Phe Asn Lys Asn Glu Ser Ile Pro Thr Ile
225                 230                 235                 240
Thr Thr Lys Ile Pro Glu Phe Asn Thr Ile Ile Gly Gln Leu Pro Asp
                245                 250                 255
Phe Ser Ala Ile Asp Leu Ile Arg Leu Asn Arg Met Tyr Asn Cys Thr
            260                 265                 270
Ala Thr His Thr Leu Leu Asp His Cys Asp Phe Glu Lys Thr Asn Val
        275                 280                 285
Cys Gly Met Ile Gln Gly Thr Arg Asp Asp Ala Asp Trp Ala His Gly
    290                 295                 300
Asp Ser Ser Gln Pro Glu Gln Val Asp His Thr Leu Val Gly Gln Cys
305                 310                 315                 320
Lys Gly Ala Gly Tyr Phe Met Phe Phe Asn Thr Ser Leu Gly Ala Arg
                325                 330                 335
Gly Glu Ala Ala Leu Leu Glu Ser Arg Ile Leu Tyr Pro Lys Arg Lys
            340                 345                 350
Gln Gln Cys Leu Gln Phe Phe Tyr Lys Met Thr Gly Ser Pro Ala Asp
        355                 360                 365
Arg Phe Glu Val Trp Val Arg Arg Asp Asp Asn Ala Gly Lys Val Arg
    370                 375                 380
Gln Leu Ala Lys Ile Gln Thr Phe Gln Gly Asp Ser Asp His Asn Trp
385                 390                 395                 400
Lys Ile Ala His Val Thr Leu Asn Glu Glu Lys Lys Phe Arg Tyr Val
                405                 410                 415
Phe Leu Gly Thr Lys Gly Asp Pro Gly Asn Ser Ser Gly Gly Ile Tyr
            420                 425                 430
Leu Asp Asp Ile Thr Leu Thr Glu Thr Pro Cys Pro Ala Gly Val Trp
        435                 440                 445
Thr Ile Arg Asn Ile Ser Gln Ile Leu Glu Asn Thr Val Lys Gly Asp
    450                 455                 460
Lys Leu Val Ser Pro Arg Phe Tyr Asn Ser Glu Gly Tyr Gly Val Gly
465                 470                 475                 480
Val Thr Leu Tyr Pro Asn Gly Arg Ile Thr Ser Asn Ser Gly Phe Leu
                485                 490                 495
Gly Leu Thr Phe His Leu Tyr Ser Gly Asp Asn Asp Ala Ile Leu Glu
            500                 505                 510
Trp Pro Val Glu Asn Arg Gln Ala Ile Met Thr Ile Leu Asp Gln Glu
        515                 520                 525
Ala Asp Thr Arg Asn Arg Met Ser Leu Thr Leu Met Phe Thr Thr Ser
    530                 535                 540

Lys Asn Gln Thr Ser Ser Ala Ile Asn Gly Ser Val Ile Trp Asp Arg
545                 550                 555                 560
Pro Ser Lys Val Gly Val Tyr Asp Lys Asp Cys Asp Cys Phe Arg Ser
                565                 570                 575
Leu Asp Trp Gly Trp Gly Gln Ala Ile Ser His Gln Leu Leu Lys Arg
            580                 585                 590
Arg Asn Phe Leu Lys Gly Asp Ser Leu Ile Ile Phe Val Asp Phe Lys
        595                 600                 605
Asp Leu Thr His Leu Asn Arg Thr Glu Val Pro Ala Ser Ala Arg Ser
    610                 615                 620
Thr Met Pro Arg Gly Leu Leu Leu Gln Gly Gln Glu Ser Pro Ala Leu
625                 630                 635                 640
Gly Glu Ser Ser Arg Lys Ala Met Leu Glu Glu Ser Leu Pro Ser Ser
                645                 650                 655
Leu Gly Gln Arg His Pro Ser Arg Gln Lys Arg Ser Val Glu Asn Thr
            660                 665                 670
Gly Pro Met Glu Asp His Asn Trp Pro Gln Tyr Phe Arg Asp Pro Cys
        675                 680                 685
Asp Pro Asn Pro Cys Gln Asn Glu Gly Thr Cys Val Asn Val Lys Gly
    690                 695                 700
Met Ala Ser Cys Arg Cys Val Ser Gly His Ala Phe Phe Tyr Ala Gly
705                 710                 715                 720
Glu Arg Cys Gln Ala Met His Val His Gly Ser Leu Leu Gly Leu Leu
                725                 730                 735
Ile Gly Cys Ile Ala Gly Leu Ile Phe Leu Thr Phe Val Thr Phe Ser
            740                 745                 750
Thr Thr Asn Gly Lys Leu Arg Gln
        755                 760

<210> 3
<211> 18
<212> PRT
<213> Artificial Sequence

<220>
<223> [CDS]:6853..6906 from SEQ ID NO 1

<400> 3
Tyr Pro Tyr Asp Val Pro Asp Tyr Ala Tyr Pro Tyr Asp Val Pro Asp
1               5                   10                  15
Tyr Ala

<210> 4
<211> 239
<212> PRT
<213> Artificial Sequence

<220>
<223> [CDS]:7590..8309 from SEQ ID NO 1

<400> 4
Met Val Ser Lys Gly Glu Glu Leu Phe Thr Gly Val Val Pro Ile Leu
1               5                   10                  15
Val Glu Leu Asp Gly Asp Val Asn Gly His Lys Phe Ser Val Ser Gly
            20                  25                  30
Glu Gly Glu Gly Asp Ala Thr Tyr Gly Lys Leu Thr Leu Lys Phe Ile
        35                  40                  45
Cys Thr Thr Gly Lys Leu Pro Val Pro Trp Pro Thr Leu Val Thr Thr
    50                  55                  60
Leu Thr Tyr Gly Val Gln Cys Phe Ser Arg Tyr Pro Asp His Met Lys

65                  70                  75                  80
Gln His Asp Phe Phe Lys Ser Ala Met Pro Glu Gly Tyr Val Gln Glu
                85                  90                  95
Arg Thr Ile Phe Phe Lys Asp Asp Gly Asn Tyr Lys Thr Arg Ala Glu
            100                 105                 110
Val Lys Phe Glu Gly Asp Thr Leu Val Asn Arg Ile Glu Leu Lys Gly
        115                 120                 125
Ile Asp Phe Lys Glu Asp Gly Asn Ile Leu Gly His Lys Leu Glu Tyr
    130                 135                 140
Asn Tyr Asn Ser His Asn Val Tyr Ile Met Ala Asp Lys Gln Lys Asn
145                 150                 155                 160
Gly Ile Lys Val Asn Phe Lys Ile Arg His Asn Ile Glu Asp Gly Ser
                165                 170                 175
Val Gln Leu Ala Asp His Tyr Gln Gln Asn Thr Pro Ile Gly Asp Gly
            180                 185                 190
Pro Val Leu Leu Pro Asp Asn His Tyr Leu Ser Thr Gln Ser Ala Leu
        195                 200                 205
Ser Lys Asp Pro Asn Glu Lys Arg Asp His Met Val Leu Leu Glu Phe
    210                 215                 220
Val Thr Ala Ala Gly Ile Thr Leu Gly Met Asp Glu Leu Tyr Lys
225                 230                 235